# EUROPEAN PATENT SPECIFICATION

(11) **EP 2 063 908 B1**
(45) Date of publication and mention of the grant of the patent: **02.10.2013**
(21) Application number: 07801920.5
(22) Date of filing: 28.08.2007
(51) Int. Cl.: A61K 38/13, A61K 38/20, C07K 14/54, A61P 37/06, A61P 39/00

(54) **USE OF MODIFIED INTERLEUKIN-8 PROTEINS FOR TREATING REPERFUSION INJURY OR TRANSPLANT REJECTION**
VERWENDUNG VON MODIFIZIERTEN INTERLEUKIN-8-PROTEINEN ZUR BEHANDLUNG VON REPERFUSIONSSCHÄDEN ODER TRANSPLANTATABSTOSSUNG
UTILISATION DE PROTÉINES MODIFIÉES DE L'INTERLEUKINE 8 POUR TRAITER LES LÉSIONS DE REPERFUSION ET LES REJETS DE GREFFE

(30) Priority: 29.08.2006 EP 06450117
(43) Date of publication of application: 03.06.2009
(73) Proprietor: ProtAffin Biotechnologie AG, 8020 Graz (AT)
(72) Inventor: KUNGL, Andreas, J., 8045 Graz (AT); BEDKE, Jens, 69115 Heidelberg (DE); GRÖNE, Hermann-Josef, 69118 Heidelberg (DE)
(74) Representative: Redl, Gerda
(86) International application number: PCT/EP2007/007497
(87) International publication number: WO 2008/025507

(56) References cited:
- WO-A-2004/058189
- WO-A-2005/054285
- US-A1- 5 665 346
- CUGINI D. ET AL.: "Inhibition of the chemokine receptor CXCR2 prevents kidney graft function deterioration due to ischemia/reperfusion" KIDNEY INTERNATIONAL, vol. 67, 2005, pages 1753-1761, XP002421838 cited in the application
- KUSCHERT G S V ET AL: "Identification of a glycosaminoglycan binding surface on human interleukin-8" BIOCHEMISTRY, AMERICAN CHEMICAL SOCIETY. EASTON, PA, US, vol. 37, no. 32, 11 August 1998 (1998-08-11), pages 11193-11201, XP002273720 ISSN: 0006-2960
- WILLIAMS GLYN ET AL: "Mutagenesis studies of interleukin-8: Identification of a second epitope involved in receptor binding" JOURNAL OF BIOLOGICAL CHEMISTRY, AMERICAN SOCIETY OF BIOLOCHEMICAL BIOLOGISTS, BIRMINGHAM,, US, vol. 271, no. 16, 1996, pages 9579-9586, XP002410539 ISSN: 0021-9258

### SEQUENCE LISTING

<110> ProtAffin Biotechnologie AG
<120> use of modified interleukin-8 proteins
<130> R 50534
<150> EP 06450117.4
   <151> 2006-08-29
<160> 2
<170> PatentIn version 3.4
<210> 1
   <211> 66
   <212> PRT
   <213> Artificial
<220>
   <223> Modified IL-8 protein
<400> 1
   Cys Gln Cys Ile Lys Thr Tyr Ser Lys Pro Lys His Pro Lys Lys Ile
   1               5                   10                  15
   Lys Glu Leu Arg Val Ile Glu Ser Gly Pro His Cys Ala Asn Thr Glu
               20                  25                  30
   Ile Ile Val Lys Leu Ser Asp Gly Arg Glu Leu Cys Leu Asp Pro Lys
           35                  40                  45
   Glu Asn Trp Val Gln Arg Val Val Glu Lys Phe Leu Lys Arg Ala Lys
       50                  55                  60
   Lys Ser
   65
<210> 2
   <211> 64
   <212> PRT
   <213> Artificial
<220>
   <223> Modified IL-8 protein
<220>
   <221> MISC_FEATURE
   <222> (1)..(1)
   <223> is SAKELR or no amino acid residue
<220>
   <221> MISC_FEATURE
   <222> (2)..(2)
   <223> is CQCI or no amino acid residue
<220>
   <221> MISC_FEATURE
   <222> (9)..(9)
   <223> is F, R, K, H, N and/or Q
<220>
   <221> MISC_FEATURE
   <222> (13)..(13)
   <223> is F, R, K, H, N and/or Q
<220>
   <221> MISC_FEATURE
   <222> (62)..(62)
   <223> is E, R, K, H, N and/or Q
<220>
   <221> MISC_FEATURE
   <222> (63)..(63)
   <223> is R, K, H, N and/or Q
<400> 2
   Xaa Xaa Lys Thr Tyr Ser Lys Pro Xaa His Pro Lys Xaa Ile Lys Glu
   1               5                   10                  15
   Leu Arg Val Ile Glu Ser Gly Pro His Cys Ala Asn Thr Glu Ile Ile
               20                  25                  30
   Val Lys Leu Ser Asp Gly Arg Glu Leu Cys Leu Asp Pro Lys Glu Asn
           35                  40                  45
   Trp Val Gln Arg Val Val Glu Lys Phe Leu Lys Arg Ala Xaa Xaa Ser
       50                  55                  60

## Description

The present invention relates to a new use of interleukin 8 (IL-8) mutant proteins having increased GAG binding affinity and further knocked-out or reduced GPCR activity compared to wild type IL-8 for the prevention and treatment of ischemic reperfusion injury and/or transplant rejection in patients. A combination preparation comprising the modified IL-8 and cyclosporin is also disclosed.

Allograft rejection is a complex interaction of inflammatory and immunologic processes that can result in hyperacute, acute, or chronic rejection and associated damage to the donor tissue. Re-jection can be considered a complex and redundant response to grafted tissue. Generally hyperacute rejection tends to be caused by existing recipient alloantibodies and is accompanied by a large number of neutrophils migrating into the graft. This process can occur within minutes to hours following transplantation and while neutrophil infiltration can often be seen in hyperacute rejection, it is believed that pre-formed anti-donor antibodies and proteins of the complement system are central to the process. Accelerated acute rejection refers to rejection which occurs between 1 to 5 days post transplant and is caused by the reactivation of sensitised T cells. Acute rejection typically occurs 1 to 5 weeks following transplantation but it may be expected to occur up to 3 months following transplantation. Acute rejection is a cell-mediated antigen-specific immune response caused by primary activation of T cells but humoral responses also are found. Chronic rejection is the most common cause currently for chronic renal transplant failure. Chronic rejection is diagnosed by chronic transplant dysfunction, and a gradual loss of function with accompanying proteinuria and hypertension (Joosten et al., (2005), Kidney International 68, pp 1-13). Chronic rejection develops in grafts that undergo persistant or intermittent cellular and humoral responses, resulting from recognition of alloantigens. On the tissue level, senescence conditioned by ischemia/reperfusion at the time of initial transplant may contribute to the development of chronic allograft nephropathy (CAN). It is believed within the field that the most effective and preferred method to prevent chronic renal failure is to avoid graft injury from both immune and nonimmune mechanisms along with patients being maintained in standard immunosuppressive drug regimes.

Further complicating the pathophysiology of organ transplantation rejection is the process of ischemia/reperfusion (I/R) injury (Versteilen et al., (2004), Int J Artifi Organs 27(12), 1019-1029). Ischemia reperfusion injury frequently occurs when the flow of blood to a region of the body is temporarily halted (ischemia) and then re-established (reperfusion). Ischemia reperfusion injury can occur during certain surgical procedures, such as repair of certain aortic aneurysms and organ transplantation or due to other events like stroke. Clinically ischemia-reperfusion injury is manifested by such complications as pulmonary dysfunction, including adult respiratory distress syndrome, renal dysfunction, consumptive coagulopathies including thrombocytopenia, fibrin deposition into the microvasculature and disseminated intravascular coagulopathy, transient and permanent spinal cord injury, cardiac arrhythmias and acute ischemic events, hepatic dysfunction including acute hepatocellular damage and necrosis, gastrointestinal dysfunction including hemorrhage and/or infarction and multisystem organ dysfunction (MSOD) or acute systemic inflammatory distress syndromes (SIRS). The injury may occur in the parts of the body to which the blood supply was interrupted, or it can occur in parts fully supplied with blood during the period of ischemia.

I/R injury is the damage caused to transplanted organs following reestablishment of blood flow, which may be more serious than that caused by ischemia following organ retrieval, storage and surgery. Impaired or delayed graft function occurs in up to 50% of cadaveric donor recipients (Carpenter (1995), Kidney Int (Supp 50): S40-S44). Delayed graft function (DGF) is defined by the requirement for supportive dialysis within the first 7 days following kidney transplantation. Such patients require longer hospital stays, extra cost and inconvenience of dialysis and greater difficulties in administering standard of care immunosuppressive drug regimes. Further, it is believed that there is a higher incidence of acute rejection episodes in patients with DGF (Shokes at al. (1996) J Urol 155: 1831-1840; Lu (1996) Curr Opin Nephrol Hypertens 5, 107-110) compared to those whose transplants function immediately.

I/R injury is a complex multifactorial disease process mediated by a number of oxygen radicals and pro-inflammatory mediators. After reperfusion, microvascular recovery in the organ can be delayed due to imbalances in cytokine release. For example, an imbalence may be produced between vasodilators such as PGI2 and nitric oxide, compared to vasoconstrictors such as thromboxane and endothelin. Further, release of toxic oxygen radicals such as ONOO- derived from NO by the superoxide radical has toxic effects and contributes to calcium influx with accompanying cell injury and death. Damaged tissues may release pro-inflammatory mediators such as Hageman factor (Factor XII) which result in the activation of platelets and the coagulation cascade, increased capillary permeability, neutrophil and macrophage chemotaxis and cytokine production.

Central to damage caused by I/R injury in organ transplantation is a nonspecific host inflammation consisting of neutrophils and macrophages. Endothelial cells become activated and upregulation of surface adhesion molecules including P- and E-selectin is seen. This results in an enhanced ability to attract neutrophils. Among the early proinflammatory mediators relevant to I/R injury and promoting neutrophil infiltration are chemokines. Interleukin-8 (IL-8) is a member of the CXC chemokine family and is believed to play a key role in I/R injury (Cugini et al. (2005) Kidney International 67, pp. 1753-1761; Sekido et al. (1993) Nature 365, 654-657). IL-8 has a major role in attracting neutrophils to the site of inflammation (Baggiolini et al. (1994), Adv Immunol 55, 97-179; Baggiolini et al. (1995), Int J Immunopharmacol 2, 103-108). A number of model systems for I/R injury and organ transplant rejection have been developed in rodents (Cugini et al. (2005), Kidney International 67, pp. 1753-1761; Groene et al., (1999), FASEB J. 13, 1371-1383). Rodents lack IL-8 but possess other homologues CXC chemokines, like Gro-alpha in rats, which acts through the (IL-8) receptors CXCR1 and CXCR2.

In inflammatory situations, neutrophils rolling on endothelial P-selectin will bind to IL-8 displayed on pathology-specific glycosaminoglycan (GAG) structures [species-specific HSPGs]. The presentation of IL-8 in a solid-phase haptotactic gradient on the surface of endothelial cells represents the key chemotactic gradient driving the activation of neutrophils in vivo (Middleton et al. (1997), Cell 91, 385-395; Proudfoot et al. (2003),PNAS 100(4), 1885-1890). GAG-bound IL-8 presented on the surface of endothelial cells binds to the CXCR1 and CXCR2 GPCR receptors present on the surface of neutrophils. This binding event leads to neutrophil activation and the expression of additional adhesion molecules, promoting the firm adhesion of neutrophils to the endothelium. Once adherent to the endothelium, the process of extravasation into the inflamed tissue follows and neutrophils are then capable of releasing further reactive oxygen species as well as proteolytic enzymes (Welbourn et al. (1991) ,Br J Surg 78, 651-655). Therefore, infiltration and accumulation of activated neutrophils into engrafted tissue plays a major role in the subsequent tissue damage and is implied in the organ dysfunction often seen in the first week following transplantation.

A number of approaches has been used to treat I/R injury in organ transplantation. These include development of neutralising antibodies to IL-8 tested in a rabbit model of lung reperfusion injury (Sekido et al. (1993),Nature 365, 654-657) and development of small molecule inhibitors of the IL-8 specific neutrophil GPCRs, CXCR1 and CXCR2, like the small molecule CXCR1/CXCR2 inhibitor Repertaxin tested in I/R injury in rat kidney graft models (Cugini et al. (2005) ,Kidney International 67, pp. 1753-1761).. Inhibition of IL-8 function can be accomplished in various ways, the most common one being antagonising the IL-8-dependent activation of CXCR1 and CXCR2 (IL-8-specific G-protein coupled receptors) on neutrophils by low molecular weight compounds. Repertaxin has been advanced into clinical trials in humans and is thought to reduce neutrophil infiltration into engrafted tissue by inhibiting CXCR2 activity in rats and CXCR1/2 activity in humans. Specifically, the authors claim that this compound inhibits CXCR2-coupled neutrophil activation.

Additionally, a modified chemokine protein based on RANTES which maintains GPCR antagonist activity, the so-called Met-RANTES, has been shown to be active in a rat model of I/R injury (Groene et al., (1999), FASEB J. 13, 1371-1383). There a modified RANTES chemokine specific for the chemokine receptors CCR1, CCR3 and CCR5 was tested, which are absent on neutrophils. The product contains no modifications to improve GAG binding characteristics and the N-terminal addition of Met- converts the protein into a GPCR antagonist. The key cell populations affected in the study were monocytes, to a lesser extent T-cells but no neutrophils.

Some previous investigators have shown that the synthetic pentasaccharide Fondapurinux, a selective inhibitor of Factor Xa can prevent neutrophil accumulation in animal models of kidney I/R injury (Frank et al. (2005), J Throm and Haemost 3, 531-540). While this pentasaccharide has been shown to be an antithrombotic agent, it has not been shown to inhibit IL-8 mediated neutrophil activation and infiltration. The mechanism of action for this selective Factor Xa inhibitor in this setting is unclear and while it is a marketed product for prevention of venous thromboembolism after orthopaedic surgery (tradename "Arixtra"), it must be administered at high doses in in vivo studies to show efficacy.

In view of the side effects and drawbacks of current I/R injury therapies and transplant rejection treatements, there is still a desire for improved treatment of ischemia reperfusion injury and transplant rejection.

Further, a long-term acceptance of grafted tissue in the absence of continuous immunosuppression is also a goal in human medicine.

The aim of the present invention is therefore to provide an alternative method for the treatment of ischemia reperfusion injury and transplant rejection.

The invention is based on the discovery that modified IL-8 having increased GAG binding affinity and inhibited or down-regulated further biological activity compared to the respective wild type IL-8 can be used for the treatment of ischemia reperfusion (I/R) injury and/or transplant rejection. Surprisingly, the modifications introduced into IL-8 are also active in the presence of anticoagulants, e.g. heparin.

The inhibited or down-regulated activity is at least a reduction or complete lack of neutrophil activation by GPCR activation.The IL-8 mutants have been designed and shown so as to NOT bind/activate CXCR1 and CXCR2. They are designated as proteinbased glycosaminoglycan antagonists since they act on the so-called co-receptors of IL-8, namely the proteoglycans which bind the chemokine via their GAG chains. Therefore, the IL-8 mutants disclosed here do not share anything with compounds like Repertaxin (Cugini et al.) except the effect of preventing IL-8-induced neutrophil migration into inflamed tissues like in the context of ischemia-reperfusion injury.

Although it has long been established that IL-8 induced neurophil infiltration is significant in I/R injury and acute rejection due to stroke or following organ transplantation, it has not been reported or suggested that said modified IL-8, a "protein-based GAG antagonist" would have such efficacy in I/R injury, esp. in kidney transplant and acute kidney transplant rejection.

Subject matter of the present invention is therefore to provide a modified interleukin 8 (IL-8) having increased GAG binding affinity and inhibited or down-regulated GPCR binding activity compared to the respective wild type IL-8 for use in the prevention and/or treatment of ischemic reperfusion injury and/or transplant rejection in patients,
wherein the amino acid sequence of said modified IL-8 molecule is
(X1)ₙ(X2)ₘ KTYSKP (X3) HPK (X4) IKELRVIES GPHCANTEII VKLSDGRELC LDPKENWVQR VVEKFLKRA (X5) (X6) S (SEQ ID No. 2)
wherein X1 is of amino acid sequence SAKELR,
wherein X2 is of amino acid sequence CQCI,
wherein X3 is selected of the group consisting of F, R, K, H, N and/or Q, preferably it is K,
wherein X4 is selected of the group consisting of F, R, K, H, N and/or Q, preferably it is K
wherein X5 is selected of the group consisting of E, R, K, H, N and/or Q, preferably it is K,
wherein X6 is selected of the group consisting of R, K, H, N and/or Q, preferably it is K,
and wherein n and/or m can be either 0 or 1.

Another object of the invention is the use in a method for inhibiting rejection of a transplant organ in a patient comprising administering to the recipient's donor, prior to transplantation, an effective amount of said modified IL-8.

According to the present invention the use in the treatment of a hospitalized patient undergoing surgical repair of a thoracic aortic or suprarenal aortic aneurysm comprising administering to said person an amount of said modified IL-8 effective to reduce or prevent ischemia-reperfusion injury is also claimed.

A further object of the invention is to provide a pharmaceutical composition comprising said modified IL-8 in combination with cyclosporin or a metabolite or synthetic analogue thereof.

The modified IL-8. molecule as used in the present invention is modified in the GAG (glycosaminoglycan) binding region leading to increased affinity towards GAG, preferably the IL-8-specific GAG ligand. Modification can be either in the naturally occurring GAG binding region or, alternatively, a new GAG binding region can be introduced in said molecule resulting in increased affinity towards GAG. By substituting at least one naturally occurring amino acid against an amino acid, preferably a basic aa, and/or substituting at least one bulky and/or acidic amino acid in the GAG binding region, an artificial and/or improved GAG binding site is introduced in said protein. By this means, an overall more electronegative molecular character can be introduced into the chemokine.

The main purpose is to increase the relative amount of basic amino acids, preferably Arg, Lys, His, Asn and/or Gln, compared to the total amount of amino acids in said site, whereby the resulting GAG binding site should preferably comprise at least 3 basic amino acids, still preferred 4, most preferred 5 amino acids. This leads to a chemokine-based GAG antagonist competing wtIL-8 off from its HSPG co-receptor.

The term "bulky amino acid" refers to amino acids with long or sterically interfering side chains; these are in particular Trp, Ile, Leu, Phe, Tyr. Preferably, the GAG binding site on the chemokine is free of bulky amino acids to allow optimal induced fit by the GAG ligand. Advantageously, positions 17, 21, 70, and/or 71 in IL-8 are substituted by Arg, Lys, His, Asn and/or Gln. Most preferred, all four positions 17, 21, 70 and 71 of IL-8 are substituted by Arg, Lys, His, Asn and/or Gln, preferably by Lys.

The modified IL-8 as used in the present, invention comprises also an inhibited or down-regulated GPCR binding activity.

The term "further biologically active region" defines a biologically active region which is not directly the GAG binding region but which may overlap with it and which, however, binds to other molecules, cells or receptors. By modifying this further biologically active region the further biological activity of this protein is inhibited or down-regulated and thereby an "inactive" protein is provided. In the case of IL-8, the biologically active region refers at least to GPCR binding. Inactivation by protein engineering according to the present invention therefore leads to an IL-8 molecule which is reduced in promoting neutrophil activation or incapable of promoting neutrophil activation.

This means for the entire approach, that on the one hand the GAG binding affinity is higher than in the wild-type GAG binding protein, so that the modified protein will to a large extent bind to the GAG instead of the wild-type protein. On the other hand, the GPCR binding activity of the wild-type protein which mainly occurs when the protein is bound to GAG, is inhibited or down-regulated, since the modified protein will not carry out this specific activity or carries out this activity to a lesser extent.

Said GPCR binding region can be modified by deletion, insertion, and/or substitution, for example with alanine, a sterically and/or electrostatically similar residue. It is possible to either delete or insert or substitute at least one amino acid in said GPCR binding region.

In the used modified IL-8 said GPCR binding region is located within the first 10 N- terminal amino acids. The first N-terminal amino acids are involved in leukocyte activation, whereby in particular Glu-4, Leu-5 and Arg-6 were identified to be essential for receptor binding and activation. Therefore, either these three or even all first 10 N-terminal amino acids can be modified by substitution, deletion or insertion in order to inhibit or down-regulate the receptor binding and activation.

For example, the modified IL-8 can have the first 6 N- terminal amino acids deleted. As mentioned above, this mutant will not or to a lesser extent bind and activate leukocytes and/or promote neutrophil activation, so that it is particularly suitable for the treatment of organ transplant rejection.

Preferably, the modified IL-8 is selected from the group consisting of del6F17RE70KN171R, del6F17RE70RN71K, del6E70KN71K, del6F17RE70RN71K, and del6F17KF21KE70KN71K.

The amino acid sequence of the modified IL 8 molecule is described by the general formula:
(X1)ₙ(X2)ₘ KTYSKP (X3) HPK (X4) IKELRVIES GPHCANTEII VKLSDGRELC LDPKENWVQR VVEKFLKRA (X5) (X6)S (SEQ ID No. 2)

wherein X1 is of amino acid sequence SAKELR,
wherein X2 is of amino acid sequence CQCI,
wherein X3 is selected of the group consisting of F, R, K, H, N and/or Q, preferably it is K,
wherein X4 is selected of the group consisting of F, R, K, H, N and/or Q, preferably it is K
wherein X5 is selected of the group consisting of E, R, K, H, N and/or Q, preferably it is K,
wherein X6 is selected of the group consisting of R, K, H, N and/or Q, preferably it is K,
and wherein n and/or m can be either 0 or 1.

In a preferred embodiment the sequence of the modified IL-8 molecule is as follows:
CQCI KTY SKPKHPKKIK ELRVIESGPH CANTEIIVKL SDGRELCLDP KENWVQRVVE KFLKRAKKS (SEQ ID No. 1).

Preferably, the modified IL-8 is similar or identical to modified IL-8 as disclosed in WO 05/054285.

An administration schedule for the modified IL-8 is proposed where the initial dose is administered i.v. to the transplant recipient either just before organ reperfusion or immediately after organ reperfusion.

The medicament can be administered within 72 hours, alternatively within 48 hours, alternatively within 24 hours, alternatively immediately before and/or after a surgical intervention in case of organ, tissue or cell transplantation.

It has been shown in lung I/R injury that IL-8 release is maximal within 1 hour after reperfusion (De Perrot et al. (2002), Am J Respir Crit Care Med 165, 211-215). It has also been shown that mRNA expression of Gro-alpha, the rat homolog of IL-8 is maximal at 3 hours after ischemia/reperfusion (Cugini et al. (2005), Kidney International 67, pp. 1753-1761). Further, renal levels of Gro-alpha measured by ELISA reached a peak at 3 hours after reperfusion and decreased gradually thereafter (Mizutani et al. (2000), Blood 95: 3781-3787). Kinetic studies have shown that the neutrophil accumulation in the kidney following I/R injury is maximal at 24 hours (Okusa (2002) ,Nephron 90, 133-138). It is believed that significant neutrophil accumulation may still occur as late as 48 hours following reperfusion. Although leukocyte infiltration in response to I/R injury is well described, there are reports of potential beneficial effects on graft function and tissue injury by the prevention of granulocyte accumulation (Sekido et al. (1993), Nature 365, 654-657). A balance has to be reached between the optimal suppression of neutrophil infiltration following transplantation, and the beneficial effects of normal leukocyte trafficking for aiding tissue healing, and combating of bacterial, viral and other infections.

The administration of the active ingredients may be by intravenous, intramuscular or subcutaneous route. Other routes of administration, which may establish the desired blood levels of the respective ingredients, are also comprised.

The i.v. administration may be via a vein in the arm or via the hepatic artery which leads directly to the kidney. The optimal duration of pharmacodynamic effect is envisaged up to 72 hours, corresponding to the peak levels of both IL-8 levels and neutrophil accumulation seen in patients. Following the initial dosing at the point of organ reperfusion it is expected that subsequent doses will be given i.v. at least once per day but up to four times per day for 72 hours.

Surprisingly, modified IL-8 can also be applied in the presence of anticoagluants without any side effects.

In case the modified IL-8 is to be administered for the purposes of reducing the incidence of acute rejection episodes, it is expected that the compound will be administered at relevant regular interval, preferably daily, for up to three months. Should the modified IL-8 be administered for the purposes of reducing the incidence of chronic rejection and long-term organ dysfunction, it is expected that the compound will be administered at relevant regular interval for the duration of graft survival. The administration interval can be daily, up to a weekly interval.

The medicament can be formulated together with a pharmaceutically acceptable carrier.

"Pharmaceutically acceptable" is meant to encompass any carrier, which does not interfere with the effectiveness of the biological activity of the active ingredient and that is not toxic to the host to which it is administered. For example, for parenteral administration, the modified IL-8 may be formulated in unit dosage form for injection in vehicles such as saline, dextrose solution, serum albumin and Ringer's solution.

Besides the pharmaceutically acceptable carrier also minor amounts of additives, such as stabilisers, excipients, buffers and preservatives can be included.

According to the present invention is the use in a method for inhibiting rejection of a transplant organ in a patient comprising administering to the recipient's donor, prior to transplantation, effective amount of a modified IL-8 having increased GAG binding affinity compared to the GAG binding affinity of a respective wild type IL-8 and inhibited or down-regulated GPCR binding activity is also comprised.

The term "effective amount" refers to an amount of modified IL-8 that is sufficient to affect the course and the severity of the rejection of transplanted organs, tissues or cells, leading to the reduction or remission of such pathology. The effective amount will depend on the route of administration and the condior the patient.

The dose of compound application is preferably 0.8 mg/kg body weight, alternatively 0.4 mg/kg body weight, alternatively 1.2 mg/kg body weight.

Administration of the modified IL-8 can be by all applicable routes, like oral, by inhalation, intranasal, intravenous, intramuscular or subcutaneous administration.

In case the organ is harvested from a donor, the administration of the modified IL-8 is approx. 24 hours before organ harvest.

Ischemia reperfusion injury as treated according to the present invention can be caused by a major organ transplant, tissue or cell transplant or repair of an aneurysm. This can be for example surgical repair of a thoracic aortic aneurysm, a suprarenal aortic aneurysm, acute respiratory disease syndrome (ARDS), lung, cornea, heart, skin, liver, kidney, small intestine, or pancreas transplant, hepatic and biliary surgical resections, total or partial pancreatectomy, total and partial gastrectomy, esophagectomy, colorectal surgery, vascular surgery for mesenteric vascular disease, abdominal insufflation during laparoscopic surgical procedures, blunt or penetrating trauma to the abdomen including gun shot wounds, stab wounds or penetrating wounds or blunt abdominal trauma secondary to deacceleration injury or motor vehicle accidents, hemorrhagic shock due to blood loss, cardiogenic shock due to myocardial infarction or cardiac failure, neurogenic shock or anaphylaxis.

According to a further aspect of the invention, a method of treating a hospitalized patient undergoing surgical repair of, for example, thoracic aortic or suprarenal aortic aneurysm is disclosed comprising administering to said person an amount of modified IL-8 having increased GAG binding affinity compared to the GAG binding affinity of a respective wild type IL-8 and inhibited or down-regulated GPCR binding activity effective to reduce or prevent ischemia-reperfusion injury.

The modified IL-8 can also be combined with immunosuppressive agents. The immunosuppressive agent can be any immunosuppressant as known in the art. For example, it can be selected from the group consisting of cyclosporins or metabolites or synthetic analogues thereof, tacrolimus, rapamycin, corticosteroids, cyclophosphamide, chlorambucil, azathioprine, myclophenolate mofetil. In case cyclosporins are used, Cyclosporin A is the preferred substance.

Therefore, according to the present invention a combination of the modified IL-8 with a cyclosporin or metabolite or synthetic analogue thereof is comprised, too.

The use of cyclosporins as immunosuppressive agents is well known. Cyclosporin, a cyclic peptide, represents a group of nonpolar cyclic oligopeptides, having imnnunosuppressant activity, produced by the fungus *Tolypocladium inflatum* Gams and other fungi imperfecti. The major component, cyclosporin A, has been identified along with several other minor metabolites, cyclosporins B through N. A number of synthetic analogues have also been prepared. CSA prolongs survival of allogeneic transplants involving skin, heart, kidney, pancreas, bone marrow, small intestine and lung and is also known to suppress graft-versus-host disease (GVHD) and delayed-type hypersensitivity. Cyclosporin A is a commercially available drug, which has attained widespread clinical application as immunosuppressant in organ transplantation procedures.

High doses of cyclosporin A can cause profound and irreversible nephrotoxicity as well as hepatoxicity and cardiotoxicity. In view of the toxicity, administration of lower levels of cyclosporin A, thereby reducing the toxic effects of this agent is preferred. The preparation comprising modified IL-8 having increased GAG binding affinity and inhibited or down-regulated GPCR binding activity compared to a respective wild type IL-8 with cyclosporin can enable a reduction of the cyclosporin dosage and administration.

Alternatively, immunosuppressive agents can also be administered separately or sequentially.

The following figures and examples are describing the invention in more detail without limiting the scope of the invention.

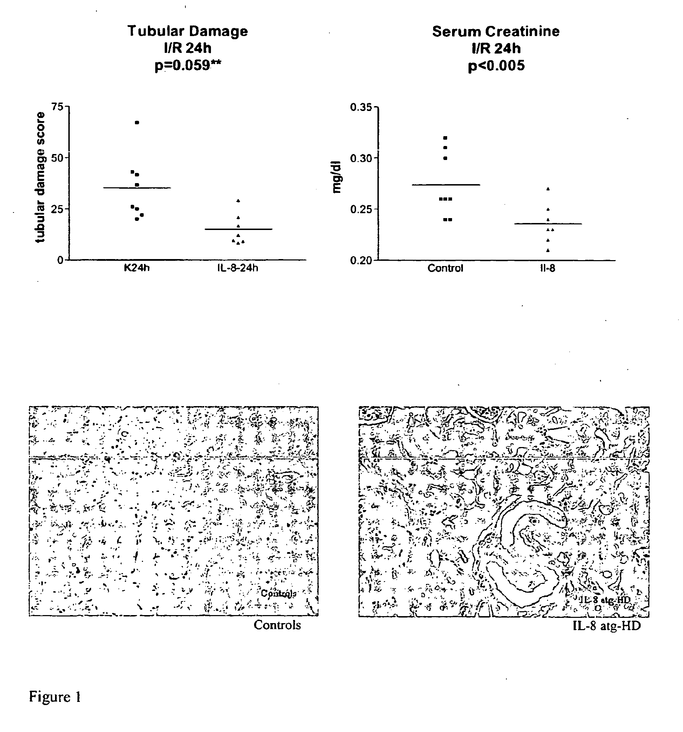

**Figure 1****:** Inhibition of ischemia/reperfusion injury in a rat model, using clamping of the renal artery for 30-40 minutes. The organ is reperfused for a further 24 hours before animals are analysed.

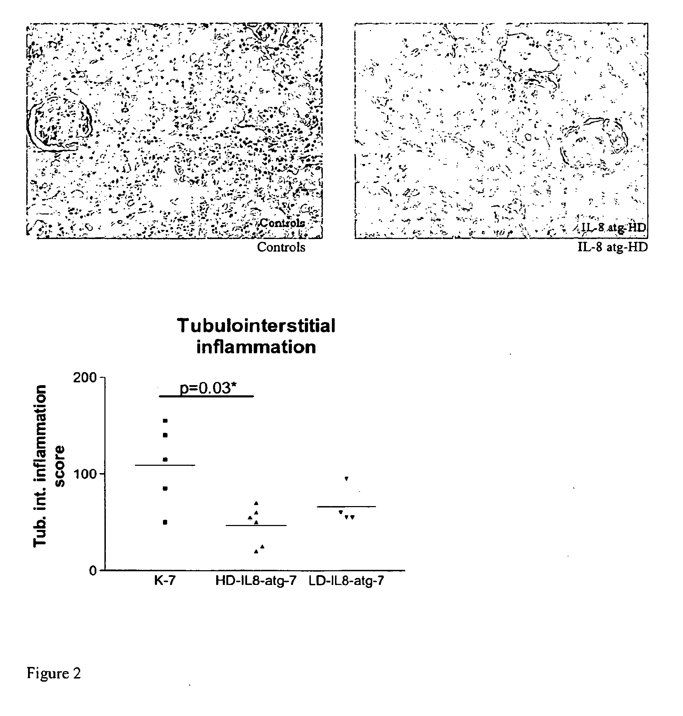

**Figure 2****:** Inhibition of acute rejection parameters following ischemia/reperfusion injury in kidney transplant. Kidneys from Fisher 3-44 rats were transplanted into Lewis rats in this moderate rejection model. Statistically significant reduction in tubulointerstitial inflammation has been shown with a reduction in leukocyte infiltration shown by histology.

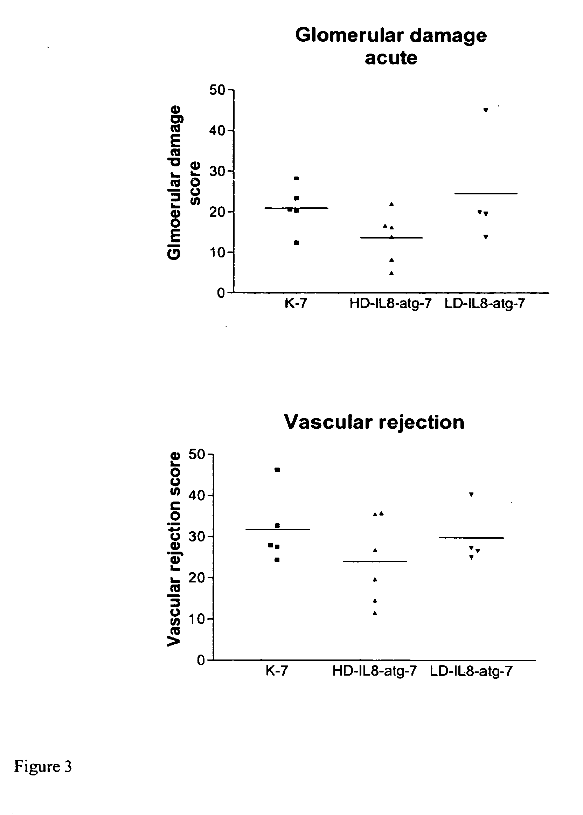

**Figure 3****:** Inhibition of acute rejection parameters following ischemia/reperfusion injury in kidney transplant. Kidneys from Fisher 344 rats were transplanted into Lewis rats in this moderate rejection model. In the same model, additional endpoints of vascular rejection score and glomerular damage score show a trend towards efficacy for the high dose treatment group.

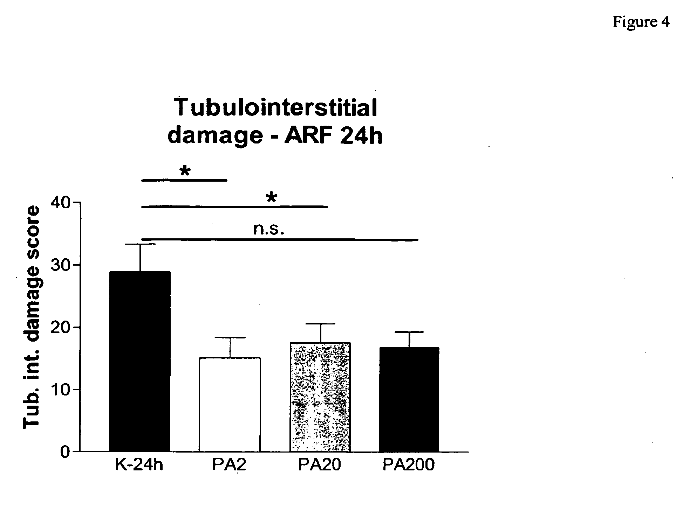

**Figure 4****:** Dose response of the del6F17KF21KE70KN71K IL-8 mutant in the rat ischemia/reperfusion injury model as measured by the tubular interstitial damage. PA2, PA20, PA200 refer to 2µg, 20µg, and 200µg of the compound.

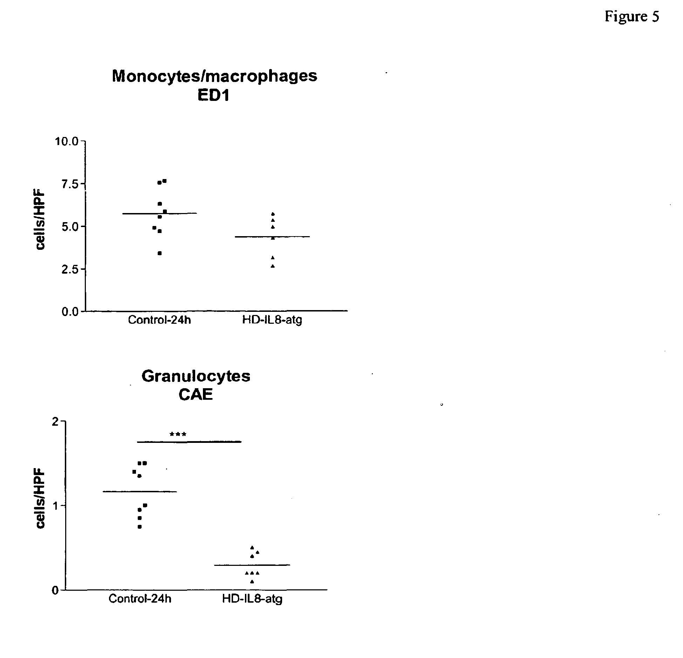

**Figure 5****:** Immunostaining of kidney tissue slices for different leukocyte sub-populations indicating a specific inhibitory effect of the del6F17KF21KE70KN71K IL-8 mutant (termed HD-IL8-atg) on granulocyte migration.

### EXAMPLES

The animal models used for the present invention were performed in the laboratories of Prof. Hermann-Josef Groene (DKFZ Heidelberg). The del6F17KF21KE70KN71K IL-8 mutant was designed as outlined previously (Potzinger et al. [2006] Biochem. Soc. Transactions 34, 435-437).

### In vitro competition experiments

Proof for the present engineering approach towards chemokinebased GAG antagonists, the proposed mode of action of the present compound in the ischemia reperfusion injury setting, comes from in vitro competition experiments. Labelling heparin, the prototypic GAG, by fluorescein provides a sensitive sensor for chemokine binding. Immobilising wtIL-8 complexed to fluorescein-tagged heparin on beads enables competition (i) with untagged heparin and (ii) with the del6F17KF21KE70KN71K IL-8 mutant. The competition constant K_{comp} for the IL-8 mutant is >100-fold improved compared to heparin which demonstrates the GAG antagonistic nature of this mutant.

### Mutant activity in the presence of anti-coagulants

Since our approach is finally targeting ischemia reperfusion injury as a first and acute immunological disorder in the context of kidney transplant, interaction with commonly applied drugs such as anti-coagulants is considered. Typically, patients are treated for this purpose with heparin so the impact of a GAG background on the action of the drug according to the invention needs to be taken into account. Performing surface plasmon resonance experiments proofs that the del6F17KF21KE70KN71K IL-8 mutant is still able to bind to heparan sulfate, the cell-surface target GAG for the present mutant proteins, in the presence of a transplant patient-like heparin background with almost identical affinity as in the absence of heparin This shows that the dominant-negative character of this protein is unaffected by the heparin background typical for transplant patients.

### Rat model of ischemia reperfusion injury preceding kidney transplant

Male Fisher rats (250 g) are taken for the kidney ischemia reperfusion experiments. Prior to surgery, anesthesia is induced with ether. After removal of the right kidney, the artery of the left kidney is dissected from the vein and clamped to induce ischemia. 45 mins after this intervention 400 µl of either buffer or compound in different concentrations are injected into the animal's penis vein. Subsequently the clip is opened and reperfusion of the organ is allowed. The rats are kept alive for further 24 hours, calculated from the time point of the reperfusion event. Again, anesthesia is induced with ether and organs (kidney, lung, heart, spleen and liver) are taken and collected in formalin solutions for histology. In addition, arterial blood is taken for the determination of creatinine concentrations in the plasma. The group size for each substance concentration was defined with four animals and the group of the control animals constituted of six.

Experimental groups were as follows:
Group 1: Six Fisher rats treated with PBS
Group 2: Six Fisher rats treated with 20µg of the del6F17K-F21KE70KN71K IL-8 mutant
Group 3: Six Fisher rats treated with 200µg of the del6F17K-F21KE70KN71K IL-8 mutant

In a second round of experiments, performed to obtain a dose-dependence of the del6F17KF21KE70KN71K IL-8 mutant in ischemia reperfusion injury experiments, the following groups were used:
Group 1: Six Fisher rats treated with PBS
Group 2: Six Fisher rats treated with 2µg of the del6F17K-F21KE70KN71K IL-8 mutant
Group 3: Six Fisher rats treated with 20µg of the del6F17K-F21KE70KN71K IL-8 mutant
Group 4: Six Fisher rats treated with 200µg of the del6F17K-F21KE70KN71K IL-8 mutant

### Rat model of moderately severe renal transplant rejection

Inbred male rats are used in all experiments. Lewis (LEW, RT11) rats serve as recipients of Fisher 344 (F344 RTllvl) kidneys. The animals are purchased from Charles River GmbH, Sulzfeld, Germany. The rats should weigh 190 to 250 gm to adjust for ureter diameter. Transplantation is performed using a modification of the technique originally described by Fisher and Lee (Fisher B. et al., 1965). Briefly, the animals are anaesthetised by ether-drop anaesthesia, the donor kidney is flushed with 5 ml of cold 0.9% NaCl (4°C) with or without compound. The kidney and ureter are removed en bloc including the renal artery with a 5-mm aortic cuff and the renal vein with a 3-mm vena cava patch. The kidneys are stored in 0.9% NaCl 4°C.

The donor kidney is transplanted to the abdominal aorta and inferior vena cava of the recipient animal, below the left renal artery, by end-to-side anastomoses with 8-0 nonabsorbable monofilament nylon suture. Ureter anastomosis is performed end-to-end with 11-0 nonabsorbable monofilament nylon suture. Total ischemic time of the donor kidney can vary between 30 and 40 min. Hyronephrosis is evaluated both macroscopically at time of death and by light microscopy. All animals with hyronephrosis are excluded from the experimental groups. The left kidney of the recipient is always removed at the time of transplantation. The right kidney is left in plate to have an internal control for the effects of the compound.

### Experimental Groups:

Experimental groups were as follows:
Group 1: Six Fisher/Lewis rats treated with PBS
Group 2: Six Fisher/Lewis rats treated with 20µg of the del6F17KF21KE70KN71K IL-8 mutant
Group 3: Six Fisher/Lewis rats treated with 200µg of the del6F17KF21KE70KN71K IL-8 mutant

### Serum analysis

Blood can be taken from the aorta at the time of sacrifice and is analysed for creatinine, urea, glucose, and bilirubin using an automated serum analyser.

Histology Organs (lung, liver, kidney, and spleen) are removed under deep anaesthesia. The organs are quickly blotted free of blood, weighed, and then processed as needed for histology, immunohistochemistry, or in situ hybridisation. The organs can be cut into 1-mm slices and either immersion-fixed in 4% formaldehyde in phosphate buffered saline (PBS) pH 7.3 5, (PB S: 99 mM NaH₂P0₄x H₂0, 108 mM NaH2P04x 2H20 and 248 MM NaCl) for 24 h or fixed in methacam for 8 h and embedded in paraffin, or frozen in liquid nitrogen and consequently stored at -80°C until used for immunohistochemistry. Light microscopy can be performed on 3 uM sections stained by periodic acid-Schiff or Goldner- Elastica.

### Immunohistochemistry

The monoclonal antibody ED1 (Serotech/Camon, Wiesbaden, Germany) has been used on methacam fixed paraffin embedded tissue (3 µM) to demonstrate monocytes/macrophages. A monoclonl antibody against chloroacetate esterase (CAE) was used to detect granulocytes/neutrophils. For detection for CD8 antigen expressed on cytotoxic T lymphocytes, monoclonal mouse antibodies are applied to frozen sections after ice cold acetone-fixation for 5 min (Serotech/Camon, Wiesbaden, Germany). An alkaline phosphatase anti- alkaline phosphatase detection system is applied (Dako, Hamburg, Germany). Controls, omitting the first or second antibody for each section tested, are supposed be negative.

### Morphometry

Vascular injury score: Preglomerular vessels with endothelial damage, thrombus and endothelialitis are assessed as showing no injury (0), a mild (1), moderate (2) and severe (3) degree of injury and evaluated in whole kidney section including cortex, outer and inner medulla. A degree specific vascular injury index is defined as the percentage of vessels with the respective degree of injury encountered in a whole kidney section. Total vascular injury score is calculated as the sum of all vessels, with all degrees of vascular injury, whereby the number of vessels with degree one, was multiplied by one, that of degree two, by a factor of two, and that of degree three, by a factor of three (Stojanovic T. et al., 1996).

Tubular inflammation score: Tubular damage is evaluated as non-existent (0), mild (1), moderate (2) and severe (3) as judged in 20 High power Fields (HPF) of cortex and cuter stripe of outer medulla. The total tubular damage score can be calculated as described for the total vascular injury score, Interstitial inflammation score: The extent of interstitial infiltration by mononuclear cells is judged as non-existent (0), mild (1), moderate (2) and severe (3) and the total score can be calculated as described for the total vascular injury score. The number of monocycles/macrophages and T cells within capillary convolutes of glomeruli is calculated as the mean of the respective numbers in all glomeruli in one kidney section.

## Claims

1. A modified interleukin 8 (IL-8) having increased GAG binding affinity and further inhibited or down-regulated GPCR binding activity compared to the respective wild type IL-8 for use in the prevention and/or treatment of ischemia reperfusion injury and/or transplant rejection in patients, wherein the amino acid sequence of said modified IL-8 molecule is (X1)ₙ(X2)ₘ KTYSKP (X3) HPK (X4) IKELRVIES GPHCANTEII VKLSDGRELC LDPKENWVQR VVEKFLKRA (X5) (X6) S (SEQ ID No. 2)
wherein X1 is of amino acid sequence SAKELR,
wherein X2 is of amino acid sequence CQCI,
wherein X3 is selected of the group consisting of F, R, K, H, N and/or Q, preferably it is K,
wherein X4 is selected of the group consisting of F, R, K, H, N and/or Q, preferably it is K
wherein X5 is selected of the group consisting of E, R, K, H, N and/or Q, preferably it is K,
wherein X6 is selected of the group consisting of R, K, H, N and/or Q, preferably it is K,
and wherein n and/or m can be either 0 or 1.

2. Modified IL-8 for use according to claim 1, **characterized in that** the GAG binding region of IL-8 is modified by substitution, insertion, and/or deletion of at least one amino acid in order to increase the relative amount of basic amino acids in said GAG binding region, and/or reduce the amount of bulky and/or acidic amino acids in said GAG binding region preferably at a solvent exposed position.

3. Modified IL-8 for use according to claims 1 or 2, **characterized in that** at least one amino acid selected from the group consisting of Arg, Lys, and His is inserted into said GAG binding region.

4. Modified IL-8 for use according to any one of claims 1 to 3, **characterized in that** positions 17, 21, 70, and/or 71 in IL-8 are substituted by Arg, Lys, His, Asn and/or Gln.

5. Modified IL-8 for use according to any one of claims 1 to 4, **characterized in that** said further biological active region is modified by deletion, insertion, and/or substitution, preferably with alanine, a sterically and/or electrostatically similar residue.

6. Modified IL-8 for use according to anyone of claims 1 to 5, **characterized in that** said modified IL-8 molecule is selected from the group consisting of del6F17RE70KN71R, del6F17RE70RN71K, del6E70KN71K, and del6F17KF21KE70KN71K.

7. Modified IL-8 for use according to anyone of claims 1 to 6, wherein the amino acid sequence of the modified IL-8 molecule is
CQCI KTY SKPKHPKKIK ELRVIESGPH CANTEIIVKL SDGRELCLDP KENWVQRVVE KFLKRAKKS (SEQ ID No. 1).

8. Modified IL-8 for use according to any one of claims 1 to 7, **characterized in that** the ischemia reperfusion injury was caused by a major organ transplant, tissue or cell transplant or repair of an aneurysm, myocardial infarction or stroke, surgical repair of a thoracic aortic aneurysm, a suprarenal aortic aneurysm, liver, kidney, small intestine, or pancreas transplant, hepatic and biliary surgical resections, total or partial pancreatectomy, total and partial gastrectomy, esophagectomy, colorectal surgery, vascular surgery for mesenteric vascular disease, abdominal insufflation during laparoscopic surgical procedures, blunt or penetrating trauma to the abdomen including gun shot wounds, stab wounds or penetrating wounds or blunt abdominal trauma secondary to deacceleration injury or motor vehicle accidents, hemorrhagic shock due to blood loss, cardiogenic shock due to myocardial infarction or cardiac failure, neurogenic shock or anaphylaxis.

9. Pharmaceutical composition comprising a modified IL-8 is defined in any one of claims 1 to 8 and a cyclosporin or a metabolite or synthetic analogue thereof.

## Patentansprüche

1. Modifiziertes Interleukin 8 (IL-8), das verglichen mit dem entsprechenden Wildtyp-IL-8 eine erhöhte GAG-Bindungsaffinität und ferner gehemmte oder herunterregulierte GPCR-Bindungsaktivität aufweist, für die Verwendung zur Verhinderung und/oder Behandlung einer Ischämiereperfusionsverletzung und/oder Transplantatabstoßung bei Patienten, wobei das modifizierte IL-8-Molekül folgende Aminosäuresequenz aufweist
(X₁)ₙ(X2)ₘ KTYSKP(X3)H P K (X4) IKELRVIES GPHCANTEII VKLSDGRELC LDPKENWVQR VVEKFLKRA (X5) (X6) S (SEQ ID NR. 2)
wobei X1 für die Aminosäuresequenz SAKELR steht,
wobei X2 für die Aminosäuresequenz CQCI steht,
wobei X3 aus der Gruppe ausgewählt ist, die aus F, R, K, H, N und/oder Q besteht, vorzugsweise steht es für K,
wobei X4 aus der Gruppe ausgewählt ist, die aus F, R, K, H, N und/oder Q besteht, vorzugsweise steht es für K,
wobei X5 aus der Gruppe ausgewählt ist, die aus E, R, K, H, N und/oder Q besteht, vorzugsweise steht es für K,
wobei X6 aus der Gruppe ausgewählt ist, die aus R, K, H, N und/oder Q besteht, vorzugsweise steht es für K,
und wobei n und/oder m für 0 oder 1 stehen können.

2. Modifiziertes IL-8 nach Anspruch 1, **dadurch gekennzeichnet, dass** die GAG-Bindungsregion von IL-8 durch Substitution, Insertion und/oder Deletion mindestens einer Aminosäure modifiziert ist, um die relative Menge basischer Aminosäuren in der GAG-Bindungsregion zu erhöhen und/oder die Menge an sperrigen und/oder sauren Aminosäuren in der GAG-Bindungsregion vorzugsweise an einer Lösungsmittel-exponierten Position zu verringern.

3. Modifiziertes IL-8 nach Anspruch 1 oder 2, **dadurch gekennzeichnet, dass** mindestens eine Aminosäure, die aus der aus Arg, Lys und His bestehenden Gruppe ausgewählt ist, in die GAG-Bindungsregion eingesetzt ist.

4. Modifiziertes IL-8 nach einem der Ansprüche 1 bis 3, **dadurch gekennzeichnet, dass** Position 17, 21, 70 und/oder 71 in IL-8 durch Arg, Lys, His, Asn und/oder Gln substituiert sind.

5. Modifiziertes IL-8 nach einem der Ansprüche 1 bis 4, **dadurch gekennzeichnet, dass** die weitere biologisch aktive Region durch Deletion, Insertion und/oder Substitution, vorzugsweise mit Alanin, einem sterisch und/oder elektrostatisch ähnlichem Rest, modifiziert ist.

6. Modifiziertes IL-8 nach einem der Ansprüche 1 bis 5, **dadurch gekennzeichnet, dass** das modifizierte IL-8-Molekül aus der Gruppe ausgewählt ist, die aus del6F17RE70KN71R, del6F17RE70RN71K, del6E70KN71K und del6F17KF21KE70KN71K besteht.

7. Modifiziertes IL-8 nach einem der Ansprüche 1 bis 6, wobei das modifizierte IL-8-Molekül die folgende Aminosäuresequenz aufweist:
CQCI KTY SKPKHPKKIK ELRVIESGPH CANTEIIVKL SDGRELCLDP KENWVQRVVE KFLKRAKKS (SEQ ID NR. 1).

8. Modifiziertes IL-8 nach einem der Ansprüche 1 bis 7, **dadurch gekennzeichnet, dass** die Ischämiereperfusionsverletzung von einem Hauptorgantransplantat, Gewebe- oder Zelltransplantat oder der Reparatur eines Aneurysmas, einem Herzinfarkt oder Schlaganfall, der chirurgischen Reparatur eines Aneurysmas der thorakalen Aorta, eines Aneurysmas der suprarenalen Aorta, einem Leber-, Nieren-, Dünndarm- oder Bauchspeicheldrüsentransplantat, hepatischen und biliären chirurgischen Resektionen, einer totalen oder partiellen Pankreatektomie, einer totalen oder partiellen Gastrektomie, einer Ösophagektomie, einer kolorektalen Operation, einer Gefäßoperation wegen mesenterischer vaskulärer Krankheit, abdominaler Insufflation bei laparoskopischen chirurgischen Verfahren, einem stumpfen oder penetrierenden Trauma des Abdomens, einschließlich Schusswunden, Stichwunden oder penetrierender Wunden, oder einem stumpfen Abdominaltrauma als Folge eines Dezelerationsverletzung oder von Fahrzeugunfällen, einem hämorrhagischen Schock wegen Blutverlust, einem kardiogenen Schock wegen Herzinfarkt oder Herzversagen, neurogenem Schock oder Anaphylaxie verursacht wurde.

9. Pharmazeutische Zusammensetzung, umfassend ein modifiziertes IL-8 nach einem der Ansprüche 1 bis 8 und ein Cyclosporin oder einen Metaboliten oder ein synthetisches Analog davon.

## Revendications

1. Interleukine 8 (IL-8) modifiée ayant une affinité de liaison aux GAG accrue et en outre une activité de liaison au GPCR inhibée ou régulée à la baisse comparé à 1'IL-8 de type sauvage respective pour une utilisation dans la prévention et/ou le traitement d'une lésion d'ischémie-reperfusion et/ou un rejet de greffe chez des patients, dans laquelle la séquence d'acides aminés de ladite molécule d'IL-8 modifiée est
(X₁)ₙ(X2)ₘ KTYSKP(X3)H P K (X4) IKELRVIES GPHCANTEII VKLSDGRELC LDPKENWVQR VVEKFLKRA (X5) (X6) S (SEQ ID NO : 2)
dans laquelle X1 a la séquence d'acides aminés SAKELR,
dans laquelle X2 a la séquence d'acides aminés CQCI,
dans laquelle X3 est sélectionné dans le groupe consistant en F, R, K, H, N et/ou Q, et est de préférence K,
dans laquelle X4 est sélectionné dans le groupe consistant en F, R, K, H, N et/ou Q, et est de préférence K,
dans laquelle X5 est sélectionné dans le groupe consistant en E, R, K, H, N et/ou Q, et est de préférence K,
dans laquelle X6 est sélectionné dans le groupe consistant en R, K, H, N et/ou Q, et est de préférence K,
et dans laquelle n et/ou m peuvent être 0 ou 1.

2. IL-8 modifiée selon la revendication 1, **caractérisée en ce que** la région de liaison aux GAG de 1'IL-8 est modifiée par substitution, insertion, et/ou délétion d'au moins un acide aminé de manière à faire augmenter la quantité relative des acides aminés basiques dans ladite région de liaison aux GAG, et/ou réduire la quantité d'acides aminés encombrants et/ou acides dans ladite région de liaison aux GAG de préférence à une position exposée à un solvant.

3. IL-8 modifiée selon les revendications 1 ou 2, **caractérisée en ce qu'**au moins un acide aminé sélectionné dans le groupe consistant en Arg, Lys, et His est inséré dans ladite région de liaison aux GAG.

4. IL-8 modifiée selon l'une quelconque des revendications 1 à 3, **caractérisée en ce que** les positions 17, 21, 70, et/ou 71 dans 1'IL-8 sont substituées par Arg, Lys, His, Asn et/ou Gln.

5. IL-8 modifiée selon l'une quelconque des revendications 1 à 4, **caractérisée en ce que** ladite autre région biologique active est modifiée par délétion, insertion, et/ou substitution, de préférence par l'alanine, un résidu stériquement et/ou électrostatiquement similaire.

6. IL-8 modifiée selon l'une quelconque des revendications 1 à 5, **caractérisée en ce que** ladite molécule d'IL-8 modifiée est sélectionnée dans le groupe consistant en del6F17RE70KN71R, del6F17RE70RN71K, del6E70KN71K, et del6F17KF21KE70KN71K.

7. IL-8 modifiée selon l'une quelconque des revendications 1 à 6, dans laquelle la séquence d'acides aminés de la molécule d'IL-8 modifiée est
CQCI KTY SKPKHPKKIK ELRVIESGPH CANTEIIVKL SDGRELCLDP KENWVQRVVE KFLKRAKKS (SEQ ID NO : 1).

8. IL-8 modifiée selon l'une quelconque des revendications 1 à 7, **caractérisée en ce que** la lésion d'ischémie-reperfusion a été provoquée par une transplantation d'organe majeur, une transplantation tissulaire ou cellulaire ou la réparation d'un anévrisme, un infarctus du myocarde ou un accident cérébral vasculaire, la réparation chirurgicale d'un anévrisme de l'aorte thoracique, un anévrisme de l'aorte surrénale, une transplantation du foie, des reins, de l'intestin grêle ou du pancréas, des résections chirurgicales hépatiques et biliaires, une pancréatectomie totale ou partielle, une gastrectomie totale et partielle, un oesophagectomie, une chirurgie colorectale, une chirurgie vasculaire pour une maladie vasculaire mésentérique, une insufflation abdominale durant des interventions chirurgicales laparoscopiques, un traumatisme contendant ou pénétrant au niveau de l'abdomen y compris des blessures par balle, une blessure par arme blanche ou une blessure pénétrante ou un traumatisme abdominal contendant secondaire à un traumatisme de décélération ou à un accident de véhicule à moteur, un choc hémorragique dû à une perte de sang, un choc cardiogénique dû à un infarctus du myocarde ou une insuffisance cardiaque, un choc neurogénique ou une anaphylaxie.

9. Composition pharmaceutique comprenant une IL-8 modifiée selon l'une quelconque des revendications 1 à 8 et une cyclosporine ou un métabolite ou un analogue synthétique de celle-ci.
